# EUROPEAN PATENT APPLICATION

(11) **EP 0 999 270 A1**
(43) Date of publication of application: **10.05.2000**
(21) Application number: 98402517.1
(22) Date of filing: 09.10.1998
(51) Int. Cl.: C12N 15/12, A61K 38/21, A61K 38/18, A61K 48/00

(54) **Method of regulating SMN gene expression**

(71) Applicant: INSTITUT NATIONAL DE LA SANTE ET DE LA RECHERCHE MEDICALE (INSERM), 75654 Paris Cédex 13 (FR); Institut Curie, 75248 Paris Cedex 05 (FR); ASSOCIATION FRANCAISE CONTRE LES MYOPATHIES, F-75013 Paris (FR)
(72) Inventor: Beretta, Laura, 75016 Paris (FR); Baron, Sophie, 92160 Antony (FR)
(74) Representative: Becker, Philippe

(57) **Abstract**

The invention relates to methods and compositions for regulating the expression of the survival motor neuron gene. The invention also relates to nucleic acid constructs that can be used to regulate gene expression in cells, in vitro, ex vivo or in vivo. The invention is particularly concerned with the use of compounds capable of increasing the levels of an IRF or Stat factor within a cell, such as interferons or neurotrophic factors, for the preparation of a composition for the regulation of SMN and/or SMNc gene expression. The invention can be used to produce various proteins or peptides, as well as for the treatment of SMA.

## Description

The present invention relates to methods and compositions for regulating the expression of the survival motor neuron gene and its copy, respectively SMN and SMNc. The invention also relates to nucleic acid constructs that can be used to regulate gene expression in cells, in vitro, ex vivo or in vivo. The invention also discloses methods of using compounds to modulate expression of SMN and SMNc in cells as well as to methods of inducing resistance against viral infection. The invention also describes antibodies against the SMN protein having advantageous properties.

Spinal muscular atrophy (SMA) is a common recessive disorder, characterized by degeneration of motor neurons of the spinal cord. Deletions or intragenic mutations on chromosome 5q13 have been implicated as the cause of SMA. The survival motor neuron ("SMN") gene has been localized to human chromosome 5q13, isolated and characterized as the disease-causing gene in SMA¹⁻⁴. The SMN gene is duplicated with an almost identical centromeric copy, SMNc. SMN is deleted or mutated in SMA patients whereas SMNc remains intact. Furthermore, the relative amount of SMNc is dramatically reduced in the most severe form termed SMA type-I, as compared to mild form of SMA termed type-III, suggesting a direct correlation between the level of SMNc and the clinical severity of the disease^{5a, 5b}.

Diagnostic methods of the SMA disease have been described using nucleic acid probes specific for the SMN gene. Approaches to the treatment of SMA have also been proposed based on the delivery of the SMN gene to affected subjects. The present invention now relates to novel methods and compositions towards therapy for SMA. The present invention also provides methods and compositions for regulating gene expression.

More particularly, the inventors have undertaken an analysis of the regulation of SMN genes, in order to elucidate the genesis and regulation of SMA and therefore with the aim of providing novel approaches to therapy of SMA. In this respect, the inventors have discovered that SMN and SMNc gene expression could be regulated in vivo, in vitro or ex vivo, providing novel approaches for regulating SMN and SMNc levels in vivo, in particular within cells of affected subjects having a deficient SMN gene expression. Furthermore, the inventors have identified, isolated and characterized a nucleic acid region, located in the 5 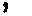 non-coding region of the SMN gene, which is a candidate Interferon-Stimulating Response Element (ISRE)⁷, overlapping with an Interferon Regulatory Factor-1 binding sequence motif (IRF-E)⁸. This region encompasses nucleotide positions between 224 and 212 bp upstream from the putative ATG translation start site of SMN, and comprises the sequence of SEQ ID NO:10. Moreover, the inventors have now demonstrated that the SMN and SMNc genes are positively regulated (i.e., stimulated) in the presence of compounds that bind or induce binding to said nucleic acid region. In this respect, the inventors have now shown that interferons rapidly induce SMN and SMNc mRNA and protein expression in cells. Both SMN and SMNc were induced, in various cell types, to the same extent by interferons. In particular, in fibroblasts from severe type I and type II SMA patients, interferon-β and interferon-γ induced SMNc gene expression and may therefore restore its expression in SMA.

The results obtained further demonstrate that several transcription factors can be involved in the regulation of SMN genes and, more preferably, the IRF members of the transcription factor family and the Stat members of the transcription factor family. In this regard, it has now been shown that IRF-1 binds to the above nucleic acid region of SMN and SMNc genes *in vitro*, and that surexpression of IRF-1 in cells induced expression of both genes in ransfection assays. IRF factors (in particular IRF-1) are therefore at least in part responsible for the induction of SMN and SMNc by interferons.

Furthermore, it has also been shown that Stat-1 binds to the above nucleic acid region of SMN and SMNc genes in vitro. Stat factors (in particular Stat-1) are therefore at least in part responsible for the induction of SMN and SMNc by interferons.

Accordingly, an object of the present invention resides in a method of regulating SMN gene expression in a cell comprising contacting said cell with a compound capable of increasing the levels of an IRF factor within a cell. Another object of this invention relates to the use of a compound capable of increasing the levels of an IRF or Stat factor within a cell, for the regulation of SMN and SMNc gene expression, in vivo, ex vivo or in vitro. Still according to the invention, is provided the use of a compound capable of increasing the levels of an IRF or Stat factor within a cell, for the preparation of a composition for the regulation of SMN and SMNc gene expression, in vivo, ex vivo or in vitro.

More preferably, the present invention resides in the discovery that interferons are capable of stimulating SMN and SMNc gene expression in a cell, that said stimulation involves a transcription factor of the IRF or Stat families, and that said stimulation is sufficient to restore normal levels of SMN protein in cells of human subjects affected with SMA.

In this regard, the invention further relates to a method of treating SMA comprising administering to a subject in needs thereof an effective amount of a compound capable of increasing the levels of an IRF or Stat factor within a cell. The invention also relates to a method of treating SMA comprising administering to a subject in needs thereof an effective amount of an interferon or an analog thereof, or a nucleic acid encoding said interferon or analog thereof. The invention also resides in the use of a compound capable of increasing the levels of an IRF or Stat factor within a cell, in particular of an interferon or an analog thereof or a corresponding nucleic acid, for the manufacture of a composition for the treatment of SMA.

The expression "treatment of SMA" designates, within the present invention, at least the partial reduction of the disease in a subject. More particularly, this expression encompasses the correction of cells deficient in SMN gene expression. Accordingly, the method of treatment according to this invention comprises, in a particular embodiment, the restoration of SMNc gene expression in cells of said subject. The restoration can also be partial or complete, depending on the compounds used and the severity of the disease. However, it is anticipated that even partial correction of SMNc gene expression should provide for a treatment of SMA, i.e., provide benefit to SMA subjects.

IRF ("Interferon Regulatory Factor") represent a family of transcriptional activators or repressors. IRF-1 was disclosed by Fujita et al. (EMBO J. 7, (1988) 3397) and Miyamoto et al. (Cell 54 (1988) 903-913). IRF-1 gene is inducible by interferons (alpha, beta and gamma), and can function as an activator, inducing transcription from promoters containing responsive sequences (i.e., corresponding binding sequences). Other members of the IRF family includes IRF-2 (which is a transcriptional repressor), and IRF-3 to 7¹⁰.

The STAT proteins (Signal Transducers and Activators of Transcription) act as transcriptional activators in response to certain stimuli or signals, and in phosphorylated form. They are involved in cell proliferation and certain immune functions. The Stat family comprises about 7 different proteins, including Stat-1 to Stat-6, which have been isolated, characterized and sequenced (M.L. Vignais, Medecine/Science 13 (1997) 1277).

According to the present invention, a compound capable of increasing the levels of an IRF or a Stat factor within a cell is a compound that stimulates the synthesis of IRF or a Stat within the cell. More preferably, a compound according to this invention is a compound capable of increasing the levels of IRF-1 or Stat-1 within a cell. Such a compound can be for instance an interferon or any analogs thereof having the capacity to stimulate IRF-1 synthesis, or a nucleic acid construct encoding an interferon or said analogs thereof. Such a compound can also be any nucleic acid construct encoding an IRF-1 product itself. Another preferred example of a compound as defined above is, for instance, a neurotrophic factor, such as CNTF (Ciliary NeuroTrophic Factor) or PDGF (Platelet Derived Growth Factor) which have now been shown to induce SMN and SMNc gene expression by increasing the levels of a Stat factor within a cell. Another type of compounds according to this invention is a double-stranded RNA or an analog thereof.

In a particular embodiment, the invention therefore lies in the use of an interferon or an analog thereof to prepare a composition for increasing SMN and SMNc gene expression in a cell.

According to another particular embodiment, the invention lies in the use of a nucleic acid encoding an interferon or an analog thereof to prepare a composition for increasing SMN and/or SMNc gene expression in a cell.

As shown in the examples, the Applicant has now demonstrated that SMN and SMNc production is inducible by interferons. More specifically, said induction can be obtained with type I and type II interferons, and in particular with interferon alpha, beta or gamma. Interferons represent a family of related cytokines, identified for their antiviral activity, but now recognized to also have important antiproliferative and apoptotic effects^{20,21}. Interferons alpha, beta and gamma have been disclosed in detail in the prior art, and are commercially available. Also, these compounds are currently being used for clinical applications in human beings, for instance in the treatment of tumor or nervous diseases (Murray et al., Ann. J. Med. 97 (1994) 459 ; Billard et al., Blood 67 (1986) 821 ; Gutterman JU., PNAS 91 (1994) 1198 ; Panitch HS., Drugs 44 (1992) 946). These compounds, or analogs thereof, are therefore compatible with clinical use in human beings, and their dosage have been described in the art.

According to the instant invention, the term "analog" designates any compounds structurally related to interferons, which have the capacity to stimulate IRF-1 or Stat-1 synthesis in a cell. A more preferred analog of an interferon is, for instance, a mutant interferon, i.e., a polypeptide whose structure comprises amino acid changes (deletions, substitution, additions) as compared to interferons, preferably less than 10% amino acid changes, more preferably less than 5% amino acid changes. Such analogs or mutants can be obtained by any method known in the art. For instance, they can be prepared by genetic, chemical and/or enzymatic modification of an interferon. They can also be produced (i.e. synthesized) artificially (for instance using nucleic acid or amino acid synthesisers). They can also be isolated from any library or natural source, when available. The capacity of these analogs to stimulate IRF-1 or Stat-1 synthesis in a cell can be evaluated as disclosed in the Examples, by contacting cells with said compound and measuring the levels of IRF-1 or IRF-1 mRNAs (or Stat-1 or Stat-1 mRNA) within the cells. The compound is regarded as stimulating IRF-1 or Stat-1 synthesis when the compound induces a 10% (preferably a 20%, more preferably a 50%) increase in IRF-1 or Stat-1 protein or mRNA levels within the cell, as compared to untreated cells.

Furthermore, in the practice of the present invention, the interferons can be used either alone, combined together, or in combination with other factors, such as, for instance, tumor necrosis factor (in particular TNFα). Such a combination indeed acts in synergy to stimulate SMN production in cells, in particular in cells of subjects affected with severe forms of SMA. The induction factor in normal cells can be five fold and, in cells affected with SMA (i.e., having a deficient SMN expression), normal SMN levels can be restored either by interferons alone or by combinations of interferons and TNF.

While the presence of certain putative binding sites in the SMN promoter region had been reported in the literature, by computer analysis of said sequence, this invention is, to our knowledge, the first report of a regulation of SMN and SMNc gene expression. In particular, this invention demonstrates, for the first time, the presence of an active binding site for a transcription factor within the SMN gene. The invention also reports, for the first time, the positive regulation (i.e., the stimulation) of SMN and SMNc gene expression by compounds. The invention further shows that induction levels sufficient to restore normal SMN protein levels in SMA subjects can be obtained. The invention also shows that pharmaceutically acceptable compounds can be used to efficiently stimulate, either alone or in combination, SMN gene in cells. The invention therefore provides a novel approach for SMA treatment.

More generally, the instant invention can also be used to prepare genetic constructs suitable to regulate expression of any selected gene. Indeed, the Applicant has now characterized a nucleic acid sequence, that is found in the SMN gene 5' region, that provides for an interferon-induced gene expression. Accordingly, said region can be isolated or artificially synthesized, inserted in any gene expression cassette, to confer to said cassette interferon inducibility.

The instant invention therefore resides also in a method of regulating gene expression in a cell, comprising

In this regard, the invention also resides in a genetic construct comprising :
a) a first nucleic acid encoding a product,
b) a transcriptional promoter operably linked to said first nucleic acid, and
c) a second nucleic acid region comprising a sequence of SEQ ID N0:10.

More preferably, the second nucleic acid comprises a sequence of 20 nucleotides comprising SEQ ID NO:10. In this regard, said second nucleic acid can be represented by a sequence of SEQ ID NO:10 or SEQ ID NO:9, for instance. In the above genetic construct, the elements a) to c) are operably linked together so that the transcriptional promoter directs expression of the first nucleic acid and is inducible by IRF-1. Furthermore, in said genetic construct, the elements a) to c) are foreign to each other, i.e. are not found associated together or not in the above form in nature. In particular, the elements a) and b) can be heterologous to each other. In a particular embodiment, the element b) and c) have the sequence of a portion of the SMN promoter comprising SEQ ID NO:9, and element a) is heterologous thereto, i.e., does not encode a SMN protein. Said element a) can encode any product of interest (protein, peptide, RNA, etc.), in particular any polypeptide product having a therapeutic or prophylactic activity (i.e., a hormone, growth factor, cytokine, enzyme, antigenic peptide, etc.). The transcriptional promoter in b) can be any promoter functional in cells, preferably in eukaryotic cells. Furthermore, the genetic construct of this invention may also comprise a transcription termination signal, located 3' of the nucleic acid a). The genetic construct can be prepared by any known method of recombinant DNA, comprising cloning, amplifying, ligating, digesting, etc. with appropriate restriction enzymes, ligases, polymerases etc. The genetic construct is preferably a DNA (in particular cDNA), although it can also be a RNA.

In a particular embodiment of the invention, the genetic construct is comprised in a vector, such as a plasmid, cosmid, viral vector (adenoviral, retroviral, AAV, herpes vector, etc), artificial chromosome, etc.

The invention also relates to the use of a nucleic acid region comprising a sequence as defined above for conferring interferon-inducibility to a gene.

In another aspect, the invention is also concerned with a cell comprising a genetic construct or a vector as disclosed above. The cell (or cell culture) can be any cell of prokaryotic or eukaryotic origin, including bacteriae, yeast cells, fungi, plant cells, animal cells, human cells, etc. The cell is more preferably of mammalian origin, such as fibroblasts, nervous cells (astrocytes, neurons, glial cells, etc.), muscle cells, blood cells (such as lymphoblastoid cells or myeloid cells, for instance), epithelial cells, embryonic cells, etc.

In the practice of the instant invention, the cells can be contacted with the compounds according to various schemes.

In particular, when the invention is performed in vitro or ex vivo, the cells can be contacted in culture, in any appropriate device (dish, flask, plate, etc.), by incubation with the compounds. In this embodiment, the cells (usually between 10⁴ and 10⁸, preferably between about 10⁵ and about 10⁷) are preferably incubated with an amount of the compound(s) sufficient to stimulate SMN (or any other gene) expression, without exerting significant toxic effects on the cells. In a particular embodiment, when the compound is interferon, it is incubated at a dosage of between about 100 U/ml to about 10000 U/ml, preferably between about 200 U/ml to about 5000 U/ml. In a more preferred embodiment, the interferons are being used at a dose of between about 500 U/ml to about 2000 U/ml. The incubation time can vary depending on the cell number, the culture medium and condition, and the nature (and concentration) of compound used. As illustrated in the examples, the kinetic of induction by interferons using a genetic construct of this invention is usually comprised between 2 to 16 hours. It is clear that the precise conditions of the incubation can be adapted by the skilled artisan. This in vitro or ex vivo method can be used to produce any product of interest encoded by the genetic construct of the invention, or to test compounds having an activity of SMN gene function and expression, for instance.

When the invention is performed in vivo, the cells are being contacted with the compound(s) by administration of said compound(s) in vivo, for instance. In this regard, the invention also relates to a method of stimulating SMN gene expression in vivo in a subject, comprising administering to said subject an effective amount of a compound as defined above, preferably an interferon or an analog thereof. The invention also resides in a method of inducing gene expression in vivo comprising administrering to a subject (i) a genetic construct as described above in which the nucleic acid element a) comprises said gene, or a vector or a cell as defined above and (ii), an amount of a compound as defined above (preferably an interferon or an analog thereof) effective for stimulating expression of said gene. The administration can be performed according to various routes, including systemic, oral or parenteral administration, for instance. Preferred routes are intrarterial, intraveinous, intramuscular or intradermal. The dose of the compound(s) administrered can be adapted by the skilled artisan, to avoid significant toxicity to the subject.

As explained before, the present invention can be used to regulate SMN and SMNc gene expression in vivo, as a novel approach to the treatment of SMA or other related diseases. The invention is also suitable to regulate expression of any other genes introduced in the genetic construct. The invention is particularly suited for SMN gene regulation, in vitro, ex vivo or in vivo, in mammalian cells, more preferably in human cells. More specific cell types according to the invention are, for instance, astrocytes, fibroblasts, lymphoblastoid cells, myeloid cells, muscle cells or neuronal cells (motorneurons), preferably of human origin.

Moreover, the invention also discloses novel antibodies directed againt SMN protein, which can be used to measure the SMN induction level according to the invention. In this respect, the inventors have selected a particular antigenic peptide of SMN protein, and shown that this immunogen produces antibodies with advantageous properties. More particularly, to produce antibodies against SMN (polyclonal or monoclonals), a synthetic peptide derived from the N-terminal end of SMN, residues 21-35, was produced. The sequence of this particular peptide is as follows: FRRGTGQSDDSDIWD (SEQ. ID NO: 11)

This peptide was then used to immunize animals according to known methods and to produce either polyclonal antibodies (antiserum) or monoclonal antibodies (by fusion of spleen cells with myeloma cells and clonal selection). The antibodies obtained (in particular polyclonal antibodies) exhibit the capacity to bind specifically to SMN and SMNc from various species, with a good affinity and are capable of immunoprecipitating resulting complexes. These antibodies also exhibit advantageous properties for use in immunochemistry, in particular for immunostaining of tissues. Said peptide and antibodies represent further objects of this invention, as well as methods of using the same. In particular, the invention is directed at any antibody that binds to the above-defined peptide.

Other aspects and advantages of the instant invention will appear from the following detailed disclosure, which should be considered as illustrative and not limitative. All references cited in the present application are incorporated therein by reference.

### Legend to the Figures

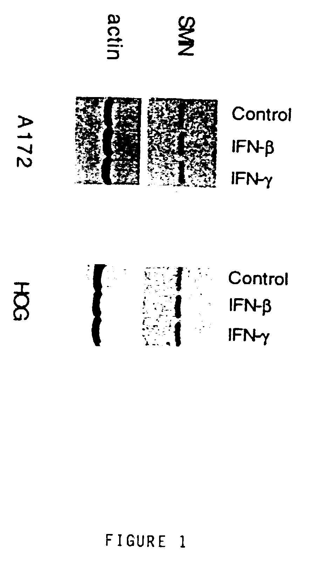

**Figure 1**. Induction of SMN protein in IFN-treated cells. A172 and HOG cells were cultured without (Control) or with 1000 U/ml of IFN-β or 1000 U/ml of IFN-γ. After 16 h of culture, cells were lysed and total protein extracts (50 µg) were analysed by Western blotting, using a polyclonal antibody against SMN (739785) (1:2000) followed by a monoclonal anti-actin antibody.

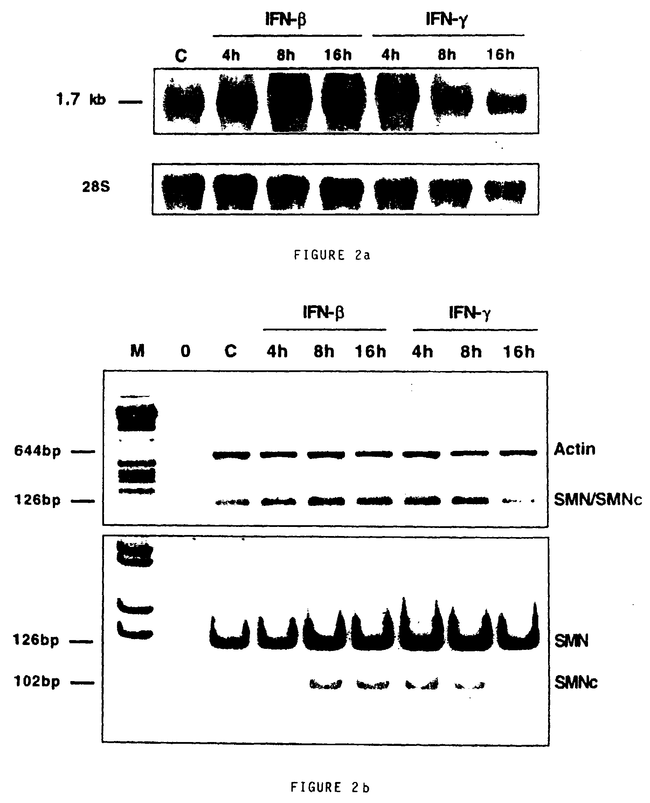

**Figure 2.** Induction of SMN and SMNc mRNAs in IFN-treated cells. (a) Northern blot analysis of RNA from untreated and IFN-treated A172 cells. A172 cells were cultured for the indicated time periods with medium alone (C) or in the presence of IFN-β (1000 U/ml) or IFN-γ (1000 U/ml). Total cellular RNA (20 µg/lane) was subjected to agarose gel electrophoresis, followed by hybridization with a (³²P)-labeled human SMN cDNA probe. Filters were subsequently hybridized to a (³²P)-labeled 28S oligonucleotide as a control of RNA loading. (b) SMN and SMNc mRNAs expression in control and IFN-treated A172 cells. Total RNA (1.5 µg) from A172 cells treated for the indicated time periods with medium alone (C) or in the presence of IFN-β (1000 U/ml) or IFN-γ (1000 U/ml) was reverse transcribed. RT-PCR was performed for SMN and actin genes as described in *Methods* and the products were analysed on a 2% agarose gel, visualized by BET staining (upper panel), or previously subjected to digestion with *Dra*I and analysed by 7% polyacrylamide gel electrophoresis (lower panel). The size of the PCR product for SMN, SMNc and actin are respectively 126 bp, 102 bp and 165 bp.

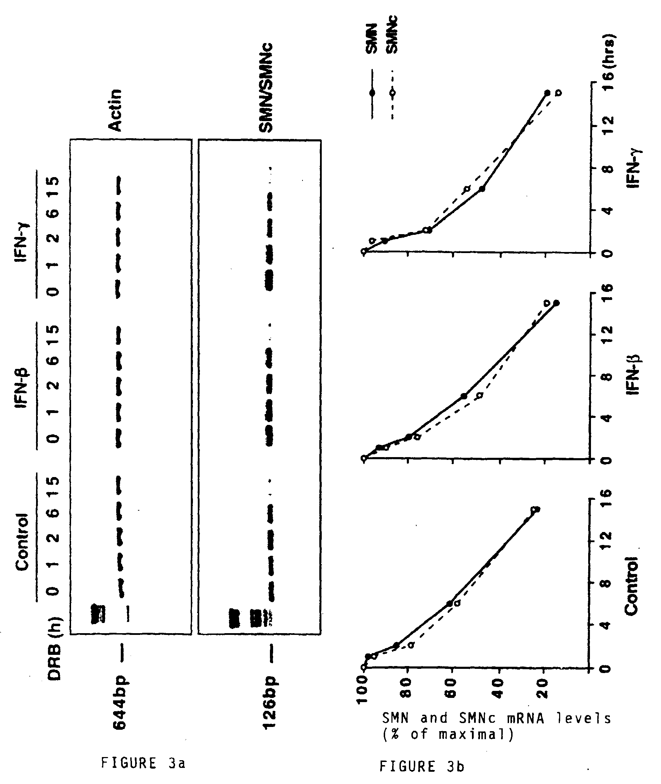

**Figure 3.** Stability of SMN and SMNc mRNAs in control and IFN-treated A172 cells. A172 cells pretreated with medium alone, IFN-β (1000 U/ml) or IFN-γ (1000 U/ml) for 8 h were treated with 25 mg/ml DRB for the indicated time periods followed by RNA extraction. RT-PCR was performed for SMN and actin genes as described in *Methods* and the products were analysed on a 2% agarose gel (a) or previously digested with *Dra*I, analyzed on a 7% polyacrylamide gel and quantified (b).

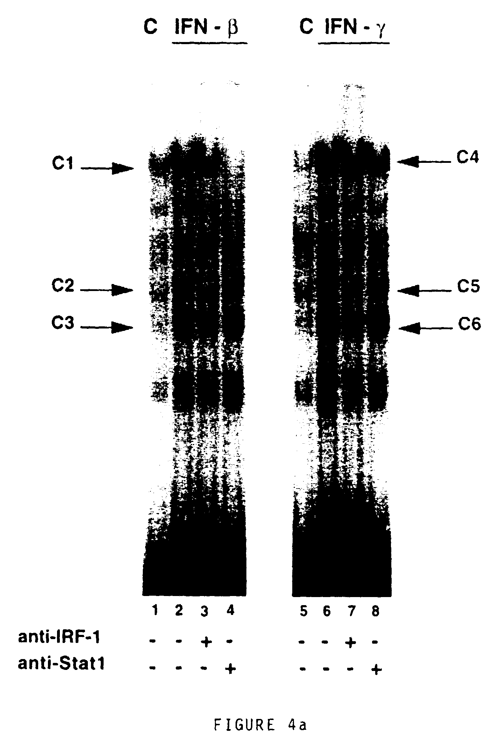

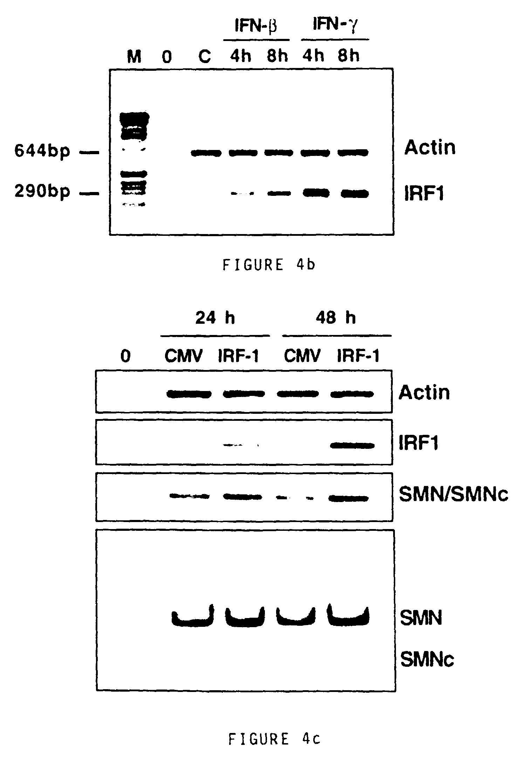

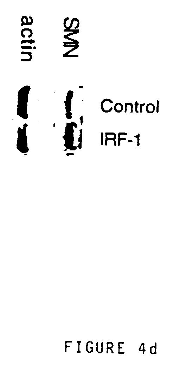

**Figure 4.** IRF-1 is involved in IFN-induced expression of SMN and SMNc genes.
   (a) Nuclear extracts prepared from untreated (lanes 1, 5), IFN-β-treated (lanes 2-4) or IFN-γ-treated (lanes 6-8) were assayed for specific binding activity to an oligonucleotide probe corresponding to SMN fragment -224 to - 202 (5'ctagaAAAGGAAAGGAAATATAACACAGt-3', SEQ ID NO: 9) after a 30 min preincubation without (lanes 1, 2, 5) or with anti-IRF-1 (lanes 3, 7) or anti-Stat1 (lanes 4, 8) antibodies. Arrows indicate C1-, C2- and C3-specific protein-DNA complexes in IFN-β-treated cells and C4-, C5- and C6-specific protein-DNA complexes in IFN-γ-treated cells.
   (b) Total RNA from A172 cells treated for the indicated time periods with medium alone (C) or in the presence of IFN-β (1000 U/ml) or IFN-γ (1000 U/ml) was reverse-transcribed and PCR coamplification was performed for IRF-1 and β-actin genes. The products were analyzed on a 2% agarose gel and visualized by BET staining. (c and d) A172 cells were cotransfected by electroporation with 16 µg of the expression plasmid pCMV/IRF-1 (generous gift from Dr. J. Hiscott) or of vector alone together with 4 µg of pEFGP vector encoding the GFP. Total cellular RNA and protein extracts were prepared at 24 h and 48 h and analyzed for SMN and SMNc expression (c) by RT/PCR analysis and (d) by Western blotting analysis. RT/PCR experiments for IRF-1 and β-actin expression were performed concurrently.

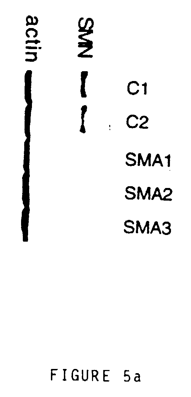

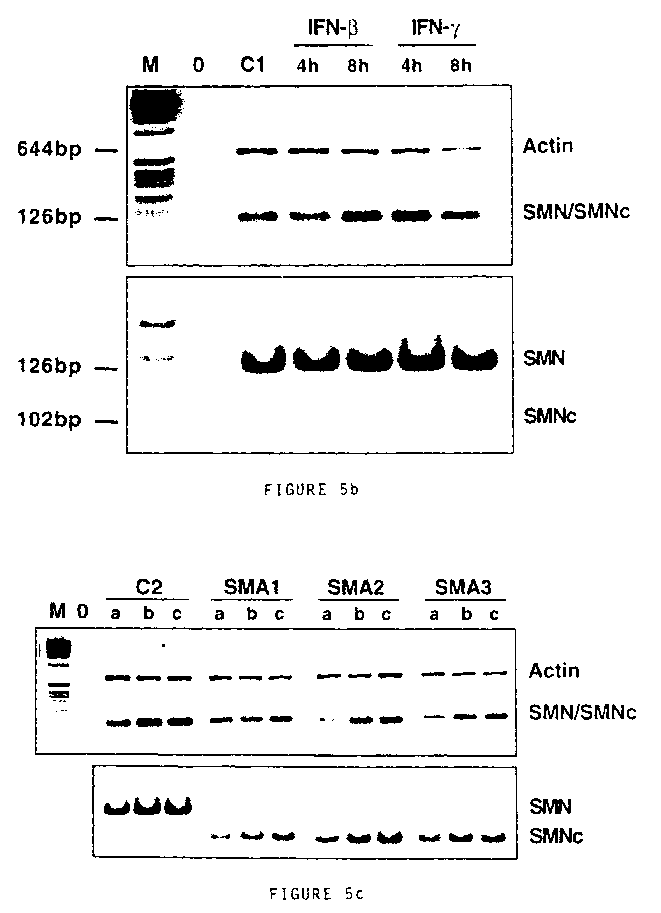

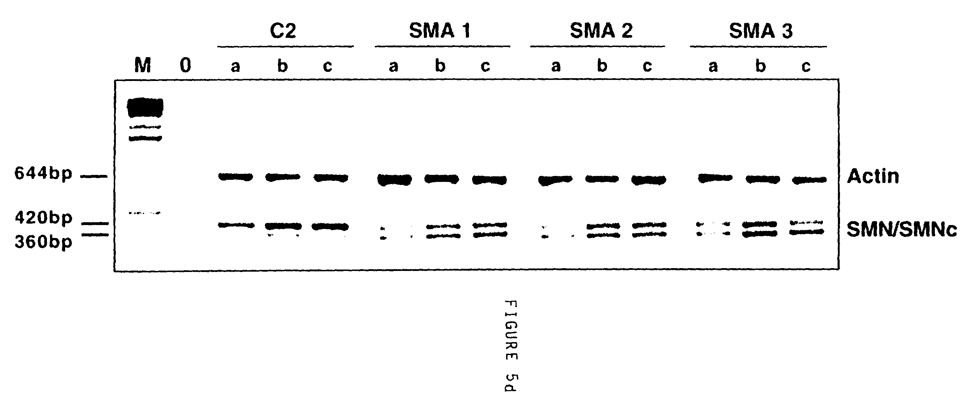

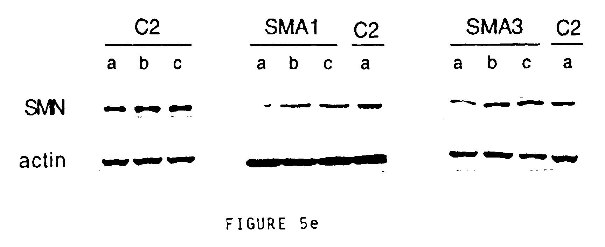

**Figure 5.** Induction of SMNc gene expression in IFN-treated fibroblasts from SMA individuals.
   (a) Total protein extracts (100 µg/lane) from fibroblasts of control (Cl, C2) and SMA patients belonging to type-I (SMA1, SMA2) or type-II (SMA3) were analyzed by western-blotting as previously described in Fig. 1. using the anti-SMN antiserum 85.
   (b) Total RNA from control fibroblasts (C1) untreated or treated for the indicated time periods with IFN-β (1000 U/ml) or IFN-γ (1000 U/ml) was reversed-transcribed and PCR coamplification was performed for SMN-SMNc and actin genes. The products were analyzed on a 2% agarose gel (upper panel) or previously digested with *Dra*I and analyzed by 7% polyacrylamide gel electrophoresis (lower panel). Lane (o) represents the negative control which was the product of PCR where RNA was omitted.
   (c) Total RNA from fibroblasts of control (C2) and SMA patients belonging to type-I (SMA1, SMA2) or to type-II (SMA3), either untreated (a), treated with IFN-β (1000 U/ml) for 8 h (b), or treated with IFN-γ (1000 U/ml) for 4 h (c), were analyzed for SMN and SMNc expression by RT/PCR experiments as described above.
   (d) RT/PCR experiments were performed using the same RNA samples but another pair of SMN-SMNc primers, designed to generate a 420-bp and a 360-bp fragment corresponding to the full-lenght transcript of SMN-SMNc and to the alternatively spliced isoform lacking exon 7 respectively.
   (e) Total protein extracts (100 µg/lane) from fibroblasts of control (C2) and SMA patients, type-I (SMA1, SMA2) and type-II (SMA3), either untreated (a), or treated for 16 h with IFN-β (1000 U/ml) (b), or with IFN-γ (1000 U/ml) (c) were analyzed by western-blotting as previously described in Fig. 1, using the anti-SMN antiserum 85.

### A - Materials and Methods

**Cells, reagents and antibodies.** The human astrocytoma cell line A172 (CRL-1620) was obtained from the American Type Culture Collection (ATCC; Rockville, MD). The human oligodendroglioma HOG cell line²² was a gift from Dr. F. Rieger. The human primary fibroblasts from SMA patients were obtained from Judith Melki. The cells were grown in Dulbecco's Minimal Essential Media (DMEM, Life Technologies, Inc.) supplemented with 10% heat-inactivated foetal bovine serum (Life Technologies, Inc.). rHuIFN-γ (specific activity, 2 x 10⁷ units/mg protein) was a gift from Roussel-Uclaf (Romainville, France); rHuIFN-β (specific activity, 4 x 10⁸ units/mg) was a gift from Ares-Serono (Geneva, Switzerland). Cells were subcultured the day preceeding IFN-treatments in order to initiate treatment of the cells in their exponential growth phase.

Rabbit affinity-purified polyclonal antibodies against human IRF-1 and Stat1 proteins were from Santa Cruz Biotechnology, Inc. (Tebu, France). To produce polyclonal antibodies against SMN, a synthetic peptide derived from the N-terminal end of SMN (residues 21-35) was used to immunize rabbits (Neosystem). The best immune response was obtained with antiserum 739785.

**Western Blotting.** Cells were lysed by successive freeze-thaw cycles, in 20 mM Tris-HCl, pH 7.5 buffer containing 5 mM EDTA and 100 mM KCl. 50 or 100 µg of protein were dissolved in sample buffer and the samples were loaded onto an SDS-10% polyacrylamide gel. Proteins were transferred onto a 0.22 µm nitrocellulose membrane (Schleicher and Schuel, Dassel, Germany), which was incubated for 2 h with rabbit polyclonal antiserum against SMN (739785) at a dilution 1:2000 followed by HRP-labeled conjugate antibody at a dilution 1:2000. Immunodetection was realized by ECL reagents and autoradiography (Amersham). The blot was rehybridized with an antibody against actin to ensure that equal amount of proteins were present in each lane.

**RNA extraction and Northern blot analysis.** Cells were scraped off directly into 4M guanidinium isothiocyanate [containing 25mM citratesodium, 0.1M 2-mercaptoethanol, Sarkosyl 0.5%, (pH7.0)], and total RNA was isolated by centrifugation through a cushion of 5.7M CsCl²³. 20 µg of RNAs were loaded on a 1.2% agarose gel with 6% formaldehyde in MOPS buffer, and then transferred onto a 0.45 µm nylon Hybond N+ membrane (Amersham). Northern blots were hybridized overnight at 42°C using radiolabelled probe (2 x 10⁶ cpm/ml). The cDNA probe for human SMN was a PCR product of 864 bp. The cDNA probes were labelled using the Redi-prime random primer labeling kit (Amersham), using (a-³²P)dCTP (DuPont NEN). After hybridization, blots were washed to high stringency and analyzed with a Phosphorlmager (Molecular Dynamics; Sunnyvale, CA). RNA was quantified and normalized by differential densitometric scanning of the SMN bands versus the 28SrRNA bands²⁴.

For determining the half-life of SMN mRNA, cells were incubated with 25 mg/ml of the RNA synthesis inhibitor 5,6-dichlorobenzimidazole riboside (DRB)²⁵ for different time periods up to 15 h before lysis in guanidium isothiocyanate as described above.

**Reverse Transcription Polymerase Chain Reaction (RT-PCR).** Total RNA was extracted as described above. Single-stranded cDNA was synthesized from the whole RNA pellet using Moloney's murine leukemia virus (M-MLV) RT and random hexamers as primers. Briefly, 1 µg RNA was mixed with 25 nmol of each dNTP (Bioprobe), 100 pmol of random hexamers (PdN6; Boehringer Mannheim, Meylan, France), 125 U of human placenta ribonuclease inhibitior (HPRI; Amersham, Les Ulis, France), and 200 U of M-MLV RT (GIBCO-BRL, Cergy-Pontoise, France) in a reverse buffer containing MgCl₂ (6.7 mmol/l), (NH₄)₂SO₄ (16.6 mmol/l), Tris HCl (67 mmol/L), pH 8.8, in a final volume of 20 µl. The mixture was incubated for 30 minutes at 42°C, heated for 5 minutes at 65°C, and stored at -80°C until used. Semiquantification of gene expression for SMN, SMNc and IRF-1 was performed after coamplification and normalization with the actin internal control sequence. Two different pairs of SMN/SMNc primers were used. The first one : 5 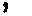 -CCTCCCATATGTCCAGATTCT-3 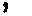 (SEQ ID NO: 1) and 5 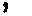 -CCTTCCTTCTTTTTGATTTTGTtT-3 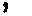 (SEQ ID NO: 2) was designed to generate a 126-bp fragment, and the second one : 5 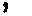 CCTCCCATATGTCCAGATTCT-3 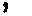 (SEQ ID NO: 3) and 5 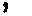 -ACTGCCTCACCACCGTGCTGG-3 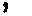 (SEQ ID NO: 4) was designed to generate a 420-bp and a 360-bp fragment depending on the alternative splicing of exon 7. The other nucleotide sequence of primers used are 5 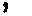 -CAACttcATCCCGGGGCTCA-3 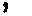 (SEQ ID NO: 5) and 5 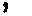 -ccctGaGGtAGCATgCGGTA-3 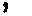 (SEQ ID NO: 6) for IRF-1 and IRF-2; 5 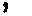 -ATCATGTTTGAGACCTTCAA-3 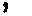 (SEQ ID NO: 7) and 5 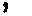 -TTGCGCTCAGGAGGAGCAAT-3 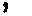 (SEQ ID NO: 8) for actin cDNA sequences. The lower case letters represent the bases included for creating restriction sites.

PCR was performed on each cDNA sample with the GeneAmp PCR System 9600 (Perkin-Elmer Corporation, Norwalk, CT); each PCR reaction was reproduced three times by sample. The reaction mixture was composed of 10 µl of cDNA template (diluted 1/50) obtained from 1 µg of extracted RNA; 50 pmol of each upstream and downstream primers for SMN or 40 pmol and 25 pmol for actin and IRF-1/IRF-2 primers respectively; 25 nmol of each dNTP (Bioprobe); 1.25 U of Taq DNA polymerase (Boehringer); 10 µl of 10xPCR buffer (17 mmol/l MgCl₂, 166 mmol (NH₄)₂SO₄, 670 mmol/l Tris HCl at pH 8.8) in a final volume of 100 µl. Preliminary experiments were performed to determine the conditions in which cDNAs were amplified in the linear region of the PCR reaction curve. Thermocycling conditions for SMN/SMNc and actin amplifications were as follows: denaturation at 94°C for 5 min, amplification during 35 cycles composed of denaturation at 94°C for 50 s, annealing at 57°C for 50 s, and extension at 72°C for 20 s, followed by a final extension at 72°C for 10 min. For IRF1/IRF2 amplification, the cycles parameters used were: three cycles of denaturing at 96°C for 30 s, annealing at 46°C for 50 s, extension at 72°C for 1 min followed by 30 cycles of denaturing at 96°C for 25 s, annealing at 60°C for 50s, extension at 72°C for 1 min and final extension at 72°C for 10 min. Negative controls were the product of RT were RNA was omitted. The PCR products were visualized on a 2% agarose gel by ethidium bromide staining.

The first pair of SMN/SMNc primers described above were used to create a unique *Dra*I restriction site, only present in the SMNc, but absent from the SMN cDNA PCR products as previously described²⁶. A unique *Eco*RI site present in the IRF-2, but absent from the IRF-1 cDNA PCR products was used as a restriction fragment length polymorphism to distinguish the amplified transcripts from the two genes as previously described²⁷. The PCR products were subjected to enzymatic digestion with *Dra*I for SMN/SMNc PCR products and *Eco*RI for IRF-1/IRF-2 PCR products. The digested products were analyzed on a 7% polyacrylamide gel allowing separation of SMN and SMNc transcripts, as well as IRF-1 and IRF-2 transcripts. The expression level for each transcript was evaluated by calculating the ratio of SMN and SMNc on actin signals after ethidium bromide staining by densitometric scanning using a video camera (Imager; Appligene, Illkirch, France) and analysis using Image 144611 MacIntosh software.

**Electromobility shift assays.** To prepare nuclear extracts, 20 x 10⁶ cells were washed twice with ice-cold PBS and then resuspended in 200 µl of buffer A (10 mM HEPES, pH 7.9, 60 mM KCl, 1 mM EDTA, 10 mM sodium orthovanadate, 0.5 mM dithiothreitol and 0.1% Nonidet P-40) supplemented with the following protease inhibitors: 1 mM phenylmethylsulfonyl fluoride and 5 mg/ml of leupeptin, aprotinin, pepstatin, antitrypsin and chymostatin. After swelling for 5 min on ice, nuclei were pelleted by centrifugation at 4000 rpm for 5 min, at 4°C. The nuclear pellet was then washed in the same buffer without Nonidet P-40 and resuspended in buffer B (20 mM Tris-HCl, pH 8, 1.5 mM MgCl₂, 600 mM Kcl, 0.2 mM EDTA, 0.5 mM dithiothreitol, 25 % glycerol and the mixture of protease inhibitors described above). After two cycles of freezing/defrosting in nitrogen liquid, nuclear debris were centrifuged at 12000 x g for 45 min at 4°C and the supernatant was aliquoted and stored at -80°C until analysis by EMSA. Protein was quantified using Bradford's assay.

For EMSA, proteins (10 µg) were incubated with radiolabeled probe (30000 cpm) in binding buffer (10 mM Tris-HCl, pH 7.4, 50 mM NaCl, 0.5 mM dithiothreitol, 18% glycerol, 0.2% Nonidet P-40 and 2 mg of poly(dl-dC)) in a total volume of 20 µl. After 30 min incubation on ice, the nucleoprotein complexes were loaded onto a 5 % non-denaturing acrylamide gel in TGE buffer (50 mM Tris-HCl, pH 8.8, 180 mM glycline and 2.5 mM EDTA) and migrated 3 hours at 20 mAmp, in refrigerated conditions. The gel was dried and exposed overnight to a Phosphorlmager screen (Molecular Dynamics, Sunnyvale, CA). For competition experiments, antisera were mixed directly with nuclear extracts and binding buffer 30 min at 4°C before adding radiolabeled probe. Purified synthetic oligonucleotides were provided by Genset (Paris, France), and covered SMN promoter fragment between -224 and -202 (5 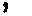 -ctagaAAAGGAAAGGAAATATAACACAGt-3 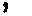 , SEQ ID NO:9). The lowercase letters represent the bases included for creating restriction sites. After annealing, the synthetic double-stranded oligonucleotides were end-labeled using the Klenow-DNA polymerase and (a-³²P)dCTP (DuPont NEN) and were purified on Elutip™ columns (Schleicher & Schuell).

**Transient Transfections.** 20 x 10⁶ cells (logarithmic growth phase) were washed two times with serum-free DMEM and resuspended in 200 ml of the same medium. Cells were then mixed gently with 16 µg of the expression plasmid pCMV/IRF-1 or of the vector alone pCMV (a generous gift from Dr. P. M. Pitha, Oncology Center, Baltimore, MD, Hiscott) together with 4 µg of the pEGFP vector encoding the green fluorescence protein (Clontech) as a monitor for transfection efficiency. After 10 min on ice, cells were electroporated at 210 V, 960 microfarads, using a Gene Pulser wired to an electroporation chamber (Bio-Rad). Cells were then immediately diluted in 10 ml of DMEM supplemented with 10% SVF and reseeded. 24 h and 48 h after transfection, cells were harvested for preparing total cell extracts and total cellular RNA. Concurrently, cells were observed microscopically and transfection efficiency was evaluated.

### B - Results

### 1. IFNs induced SMN expression.

To investigate the effects of IFNs on SMN expression, we utilized the human astrocytoma cell line, A172. Cells were treated with IFN-β (1000 U/ml) or IFN-γ (1000 U/ml) for 16 h and SMN protein expression was analyzed by western-blotting using a polyclonal antibody 739785 which we raised against a synthetic peptide of human SMN. A unique band of 38 kD specific to SMN protein was detected. The relative amount of SMN protein was increased by 3-fold in both IFN-β- and IFN-γ-treated cells, as determined by densitometric analysis (**Fig. 1, left panels**). To demonstrate that this induction was not limited to A172 cells, similar results were also obtained with the human oligodendroglioma HOG cell line treated with IFN--β and IFN-γ (**Fig. 1, right panels**). Induction of SMN by interferons was also observed in other cell types of various origin including fibroblasts (**see Fig. 5**), lymphoblastoid and myeloid cells. Induction of SMN protein by IFN-β was apparent after 16 h and was maximal after 24 h whereas induction of SMN by IFN-γ was rapid and transient as induction was observed as early as 8 h of treatment and peaked after 16 h. Therefore, SMN protein expression was induced by both type I and type II IFNs (respectively IFN-β and IFN-γ) to the same extent and in various cell types, and the response to IFN-γ was very rapid and preceded the response to IFN-β.

We performed Northern Blot analysis of SMN mRNA in A172 cells treated for various times with IFN-β (1000 U/ml) and IFN-γ (1000 U/ml). Northern blot analysis demonstrated also an upregulation of SMN mRNA after IFN-β or IFN-γ treatments with a maximum induction of 4-fold (**Fig. 2a**). As observed for SMN protein, IFN-γ induction of SMN mRNA was more rapid than with IFN-β-induction. Maximal induction of SMN mRNA occured between 8 and 12 h following IFN-β treatment whereas the induction of SMN mRNA was already maximal 4 h after treatment with IFN-γ. There was therefore a strong correlation between the induction of SMN mRNA and SMN protein levels.

### 2. IFNs induced SMN and SMNc expression to the same extent.

Western-blotting and Northern-blotting analyses of SMN do not allow to distinguish between the SMN gene and its centromeric homologue SMNc. Therefore, to discriminate between the induction of each gene by IFNs, evaluation of SMN and SMNc mRNA expression levels in control and IFN-treated A172 cells was achieved by RT-PCR experiments. In order to measure SMN and SMNc gene expression, we used an assay system based on RT/PCR coamplification of transcripts from both genes, creating a unique *Dra*I site, only present in the SMNc but absent from the SMN cDNA PCR products. Therefore digestion of the PCR products with *Dra*I allowed to distinguish the amplified transcripts from the two genes. Normalization and semiquantification of SMN and SMNc genes expression were performed by using internal control PCR with actin oligonucleotides. As shown in the upper panel of **Fig. 2b** corresponding to the undigested PCR products, induction of SMN/SMNc expression was observed in IFN-β- and in IFN-γ-treated cells, with a faster response observed with IFN-γ in agreement with the results of Northern blot analysis. Following separation of SMN and SMNc cDNA PCR products, we observed that expression of SMN and SMNc genes in control A172 cells was dramatically different (90% and 10%, respectively). Treatment with IFN-β or IFN-γ resulted in equal induction of SMN and SMNc with comparable kinetics (**Fig. 2b, lower panels**).

### 3. Stability of SMN and SMNc mRNAs.

Next we determined the contribution of SMN and SMNc mRNAs stability to the differences observed between the expression levels of the two genes, and to the induction of SMN and SMNc gene expression by IFNs. A172 cells were treated with IFN-β (1000 U/ml) or IFN-γ (1000 U/ml) for 8 h. Control and treated cells were incubated with 25 mg/ml of DRB, a potent inhibitor of RNA synthesis. In time course experiment, we observed a progressive decline in the level of SMN transcripts following treatment with DRB. Following digestion of the RT/PCR products with *Dra*I and separation of SMN and SMNc transcripts, we observed that the difference in expression in SMN and SMNc in A172 cells was not due to differential stability of the two genes as estimated half-life was of 8 h for both genes (**Fig. 3; left panel**). Furthermore, IFN-β and IFN-γ did not significantly modulate neither SMN nor SMNc mRNAs stability as this declined exhibited close kinetics in IFN-treated cells as compared to control cells (**Fig. 3)**.

### 4. IRF-1 is involved in IFN-induced SMN and SMNc expression.

To characterize the molecular basis of IFN-induction of SMN and SMNc, nuclear protein extracts, prepared from both untreated cells and cells stimulated for 2 h with IFN-β or IFN-γ were utilized for electromobility shift assays with radiolabeled double-stranded synthetic oligonucleotides encompassing the putative ISRE/IRF-E motif in the 5 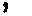 end of SMN genes. Formation of 3 major protein-DNA complexes was apparent after treatment with IFN-β (complexes C1, C2 and C3) or IFN-γ (complexes C4, C5 and C6) and the pattern of the protein-DNA complexes was similar after both treatments (**Fig. 4a; compare lanes 2 and 6**). ISRE is an element known to bind ISGF3, a heterotrimeric transcription factor consisting of Stat1 (Signal transducers and activators of transcription), Stat2 and p48 (ISGF3g)¹². IRF-E is the consensus binding site for the IRF-1 transcription factor¹³. Antibodies against IRF-1 and Stat1 were therefore used to probe the protein-DNA complexes for the presence of corresponding proteins. Addition of antisera against the IRF-1 protein impaired or strongly reduced the formation of two protein-DNA complexes induced by both IFN-β and IFN-γ, respectively C2, C3 and C5, C6 (**Fig. 4a;** compare lanes 2 and 3 and lanes 6 and 7). Addition of antisera against the Stat1 subunit impaired the formation of the upper protein-DNA complexe C1 induced by IFN-β but did not modify the upper IFN-γ-induced protein-DNA complexe C4. These results suggested that IRF-1 was part of the major complexes commonly induced by both IFN-β and IFN-γ. Stat1 was part of a complexe induced only by IFN-β whereas another complexe induced by IFN-γ involved a factor different from IRF-1 and Stat1 and still to be identified.

We investigated IRF-1 expression and induction following IFNs treatment in A172 cells by RT/PCR amplification of IRF-1 transcript (**Fig. 4b**). Expression of IRF-1 was induced by both IFN-β and IFN-γ, with a stronger effect observed following IFN-γ treatment. Furthermore, the induction of IRF-1 was maximal as early as 4 h after IFN-γ treatment whereas induction of IRF-1 was maximal after 8 h of IFN-β induction. Therefore, in A172 cells, there was a strong correlation between expression of IRF-1, SMN and SMNc genes.

### 5. IRF-1 gene delivery stimulates SMN expression

An IRF-1 expression plasmid was transfected into A172 cells as a direct approach to analyse the effect of IRF-1 on the expression of SMN and SMNc *in vivo*. The expression plasmid pCMV/IRF-1 or the vector alone were transfected together with a construct encoding the green fluorescence protein GFP as a monitor for transfection efficiency. The transfection efficiency was similar (18%) in the control pCMV- and pCMV/IRF-1 transfected cells. IRF-1, SMN and SMNc expression were analysed 24 h and 48 h post-transfection by RT-PCR amplification (Figure 4c).

As expected, IFR-1 expression was not detected in cells transfected with the vector alone. In cells transfected with the IRF-1 plasmid, IRF-1 mRNA expression was detected as early as 24 h after transfection and increased 48 h after transfection. Both SMN and SMNc gene expression were induced in cells transfected with the IRF-1 plasmid as compared to cells transfected with the vector alone. The effect was stronger 48 h than 24 h after transfection. Western blot analysis showed also an increase in SMN protein level in IRF-1 transfected cells as compared to control cells, 48 h after transfection (data not shown). Overexpression of IRF-1 is thus sufficient to trans-activate SMN and SMNc expression in these cells.

### 6. IFNs restored SMNc gene expression in fibroblasts from SMA patients.

The effect of IFNs treatment on SMNc expression was examined using primary culture of fibroblasts from type I and type II patients that exhibited markedly reduced SMN protein levels. As previously described (4), immunoblotting analysis demonstrated that both SMN and SMNc genes were expressed at the protein level with similar mobilities and that the amount of the SMNc protein was dramatically reduced in SMA type I (SMA1 and SMA2) and type II (SMA3) fibroblasts as compared to controls (C1 and C2) (Fig. 5a). As previously described in IFN-treated A172 cells, induction of SMN and SMNc gene expression was observed both in IFN-β- and IFN-γ-treated primary culture of control C1 fibroblasts, with a faster response to IFN-γ (Fig. 5b). We therefore evaluated SMN/SMNc mRNA expression by RT-PCR amplification, in control and SMA fibroblasts following 8 h of treatment with IFN-β or 4 h of treatment with IFN-γ (Fig. 5c: upper panel). In control fibroblasts, expression of SMN was dramatically stronger than expression of SMNc as already shown in A172 cells. SMNc mRNA expression was induced following treatment with IFN-β or IFN-γ, in SMA type I and type II patients. Previous reports showed that SMNc gene produces an alternatively spliced isoform lacking exon 7 in comparable amount to the full-length transcript, whereas SMN gene generates primarily full-length mRNA. Furthermore, it has been shown that this particular isoform demonstrated reduced self-association, a potential biochemical defect in SMA. Since the RT/PCR assay system we used only detects the full-length transcripts, we wished to investigate if IFNs treatment was also effective on the alternative spliced SMNc transcripts in SMA patients. RT-PCR amplification, using a pair of SMN/SMNc primers located in exons 6 and 8, was performed from control and SMA fibroblasts. As shown in Fig. 5d, expression of full-length and alternatively spliced transcripts differed between controls and SMA patients in agreement with other studies previously reported. Expression of transcripts retaining or lacking exon 7 was similarly induced following treatment with IFN-β and IFN-γ in controls and SMA patients.

### C - Discussion

In the present study, we provide evidence for an induction of SMN and SMNc genes expression by IFN-β and IFN-γ, respectively members of type-I and type-II interferons. Both SMN and SMNc genes respond to interferons to the same extent. This is in agreement with the previous report indicating that comparative sequence analysis showed no discrepancy between the 5 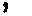 ends of the SMN gene and its centromeric counterpart SMNc⁶. More importantly, IFN-β and IFN-γ induced SMNc expression in fibroblasts from SMA patients. Therefore, the amount of SMNc expression being directly correlated to the severity of the desease, interferons appear to be a novel therapeutic approach to the treatment of SMA patients. In this respect, it has to be noted that the level of induction of SMN and SMNc gene expression by IFNs is similar to the differences of SMN expression levels observed between severe form of SMA and controls. Accordingly, this level of induction should be sufficient to restore normal SMN levels and should play an important role in SMN function.

SMN protein has been shown to bind directly to a number of proteins that participate in early spliceosome assembly, suggesting a role in RNA processing^{15,16}. SMN is present in several intranuclear dots termed "gems", closely associated with coiled bodies and nucleoli^{17,} although gems may be indistinguishable from coiled bodies in most cell types¹⁸. The evidence that SMN is an IFN-inducible gene suggest that it may play a role in the functions of interferons¹⁹. Inteferons (IFNs), represent a family of related cytokines first identified for their antiviral activity, but now recognized to also have important antiproliferative and apoptotic effects^{20,21}. Furthermore, our results demonstrate that the transcription factor IRF-1 is at least in part responsible for the induction of SMN observed following IFNs treatment. IRF-1 is the best characterized member of a growing family of Interferon Regulatory Factors (IRFs)^{10,11}. IRF-1 is expressed at low levels or is undetectable in a variety of cell types; however, its expression is inducible by virus infection, double-stranded RNA, treatment with both type I and II IFNs as well as other cytokines and activators. IRF-1 has been shown to modulate not only the cellular response to IFNs and viral infection, but also cell growth, susceptibility to transformation by oncogenes and induction of apoptosis. It would be of great interest to investigate how SMN participates in the induction of these IFN- and IRF-1 mediated cellular responses. Determination of a role of SMN in the IFN-mediated antiviral state of cells may be of considerable consequence on the determinants of the SMA disease.

### References

1. Brzustowicz, L. M., Lehner, T., Castilla, L. H. , Penchaszadeh, G. K., Wilhelmsen, K. C., Daniels, R. J., Davies, K. E., Leppert, M., Ziter, F., Wood, D., Dubowitz, V., Zerres, K., Hausmanova-Petrusewicz, I., Ott, J., Munsat, T. L. & Gilliam, T. C. Genetic mapping of chronic childhood-onset spinal muscular atrophy to chromosome 5q11.2-q13.3. *Nature* **344,**540-541 (1990).
2. Melki, J., Abdelhak, S., Sheth, P., Bachelot, M. F., Burlet, P., Marcadet, A., Aicardi, J., Barois, A., Carriere, J. P., Fardeau, M., Fontan, D., Ponsot, G., Billette, T., Angelini, C., Barbose, C., Ferriere, G., Lanzi, G., Ottolini, A., Babron, M. C., Cohen, D., Hanauer, A., Clerget-Darpous, F., Lathrop, M., Munnich, A. & Frésal J. Gene for proximal spinal muscular atrophies maps to chromosome 5q. *Nature* **344,**767-768 (1990).
3. Melki, J., Sheth, P., Abdelhak, S., Burlet, P., Bachelot, M. F., Lathrop, M., Frésal, J., Munnich, A. & the French spinal muscular atrophy invesigators. Mapping of acute (type I) spinal muscular atrophy to chromosome 5q12-q14. *Lancet* **336,**271-273 (1990).
4. Lefebvre, S., Bürglen, L., Reboullet, S., Clermont, O., Burlet, P., Viollet, L., Benichou, B., Cruard, C., Millasseau, P., Zeviani, M., Le Pasilier, D., Frézal, J., Cohen, D., Weissenbach, J., Munnich, A. & Melki, J. Identification and characterization of a spinal muscular atrophy-determining gene. *Cell* **60,**155-165 (1995).
5.
   a) Lefebvre, S., Burlet, P., Liu, Q., Bertrandy, S., Clermont, O., Munnich, A., Dreyfuss, G. & Melki, J. Correlation of severity with the SMN protein level in spinal muscular atrophy. *Nature Genetics* **16,**265-269 (1997).
   b) Coovert et al., Hum. Mol. Genet. 6 (1997) 1205-1214.
6. Bürglen, L., Lefebvre, S., Clermont, O., Burlet, P., Viollet, L., Cruaud, C., Munnich, A. & Melki, J. Structure and organization of the human Survival Motor Neurone (SMN) gene. *Genomics* **32,**479-482 (1996).
7. Darnell, J. E., Jr., Kerr, I. M. & Stark, G. R. Jak-STAT pathways and transcriptional activation in response to IFNs and other extracellular signaling proreins. *Science* **264,**1415-1421 (1994).
8. Tanaka, N., Kawakami, T. & Taniguchi, T. Recognition DNA sequences of interferon regulatory factor 1 (IRF-1) and IRF-2, regulators of cell growth and the interferon system. *Mol. Cell Biol.* **13,**4531-4538 (1993).
9. Williams, B. R. G. Transcriptional regulation of interferon-stimulated genes. *Eur. J. Biochem.* **200,**1-11 (1991).
10. Taniguchi, T., Lamphier, M. S. & Tanaka, N. IRF-1 : the transcription factor linking the interferon response and oncogenes. *Biochim. Biophys. Acta* **1333,** M9-M17 (1997).
11. Nguyen, H., Hiscott, J. & Pitha, P. M. The growing family of interferon regulatory factors. *Cytokine & Growth Factor Reviews* **8,** 293-312 (1997).
12. Schindler, C. & Darnell, J. E. Jr. Transcriptional responses to polypeptide ligands: The JAK-STAT Pathway. *Annu. Rev. Biochem.* **64,**621-651 (1995).
13. Harada, H., Fujita, T., Miyamoto, M., Kimura, Y., Maruyama, M., Furia, A., Miyata, T. & Taniguchi, T. Structurally similar but functionally distinct factors, IRF-1 and IRF-2, bind to the same regulatory elements of IFN and IFN-inducible genes. *Cell* **58,**729-739 (1989).
14. Gennarelli, M. et al. Survival motor neurons gene transcript analysis in muscles from spinal muscular atrophy patients. *Biochem. Biophys. Res. Commun.* **213,** 342-348 (1995).
15. Liu, Q., Fischer, U., Wang, F. & Dreyfuss, G. The spinal muscular disease gene product, SMN, and its associated protein SIP1 are in a complex with spliceosomal snRNP proteins. *Cell* **90,**1013-1021 (1997).
16. Fischer, U., Liu, Q. & Dreyfuss, G. The SMN-SIP1 complex has an essential role in spliceosomal sn RNP biogenesis. *Cell* **90,**1023-1029 (1997).
17. Liu, Q. & Dreyfuss, G. A novel nuclear structure containing the survival of motor neurons protein. *EMBO J.* **15,**3555-3565 (1996).
18. Matera, A. G. & Frey, M. R. Coiled bodies and Gems: Janus or Gemini? *Am. J. Hum. Genet.* **63,** 317-321 (1998).
19. Sen, G. C. & Ransohoff, R. M. Interferon-induced antiviral actions and their regulation. *Adv. Virus Res.* **42,** 57-102 (1993).
20. Pestka, S., Langer, A, J., Zoon, K. & Samuel, C. Interferons and their actions. *Annu. Rev. Biochem.* **56,**727-777 (1987).
21. De Maeyer, E. & de Maeyer-Guignard, J. Interferons and other regulatory cytokines. John Wiley and Sons, New York (1988).
22. Post, G. R. & Dawson, G. Characterization of a cell line derived from a human oligodendroglioma. *Mol. Chem. Neuropathol.* **16,** 303-317 (1992).
23. Chirgwin, J. M., Przybyla, A. E., MacDonald, R. J. & Rutter, W. J. Isolation of biologically active ribonucleic acid from sources enriched in ribonuclease. *Biochemistry* **18,** 5294-5299 (1979).
24. Barbu, V. and Dautry, F. Mevalonate deprivation alters the induction of fos and myc by growth factors. *Oncogene* **5,**1077-1080 (1990).
25. Noda, M., Yoon, K. & Rodan, G. A. Cyclic AMP-mediated stabilization of osteocalcin mRNA in rat osteoblast-like cells treated with parathyroid hormone. *J. Biol. Chem.* **263,**18574-18577 (1988).
26. Van der Steege, G., Grootscholten, P. M., Van der Vlies, P., Draaijirs, T. G., Osinga, J., Cobben, J. M., Scheffer, H. & Buys, C. H. C. M. PCR-based DNA test to confirm clinical diagnosis of autosomal recessive spinal muscular atrophy. *The Lancet* **345,** 985-986 (1995).
27. Hochhaus, A., Yan, X. H., Willer, A., Hehlmann, R., Gordon, M. Y., Goldman, J. M. & Melo, J. V. Expression of interferon regulatory factor (IRF) genes and response to interferon-a in chronic myeloid leukaemia. *Leukemia* **11,**933-939.

## Claims

1. Use of a compound capable of increasing the levels of an IRF or Stat factor within a cell, for the preparation of a composition for the regulation of SMN gene expression.

2. Use according to claim 1, wherein said compound is capable of increasing the levels of IRF-1 within a cell.

3. Use according to claim 2, wherein said compound is an interferon or an analog thereof, or a nucleic acid encoding an interferon or an analog thereof, or a combination thereof.

4. Use according to claim 3, wherein said compound is interferon alpha, beta or gamma.

5. Use according to claim 1, wherein said compound is capable of increasing the levels of Stat-1 within a cell.

6. Use according to claim 5, wherein said compound is a neurotrophic factor such as PDGF or CNTF.

7. Use according to any one of claims 3 or 4, wherein said compound or combination thereof is associated with TNFα.

8. Use of a compound capable of increasing the levels of an IRF or Stat factor within a cell, for the preparation of a composition for the treatment of Spinal Muscular Atrophy (SMA).

9. Use of an interferon or an analog thereof for the preparation of a composition for the treatment of Spinal Muscular Atrophy (SMA).

10. A genetic construct comprising
- a first nucleic acid encoding a product
- a promoter, operably linked to said first nucleic acid, and
- a second nucleic acid comprising a sequence of SEQ ID NO: 10.

11. A vector comprising a genetic construct of claim 10.

12. A cell comprising a genetic construct of claim 10 or a vector of claim 11.

13. The use of a nucleic acid comprising a sequence of SEQ ID NO: 10 for conferring interferon-inducibility to a gene.


SEQUENCE LISTING

SEQUENCE LISTING
(1) GENERAL INFORMATION:
(i) APPLICANT:
(A) NAME: INSERM
(B) STREET: 101, Rue de Tolbiac
(C) CITY: PARIS
(E) COUNTRY: FRANCE
(F) POSTAL CODE (ZIP): 75654

(i) APPLICANT:
(A) NAME: INSTITUT CURIE
(B) STREET: 26, Rue d'Ulm
(C) CITY: PARIS
(E) COUNTRY: FRANCE
(F) POSTAL CODE (ZIP): 75248

(i) APPLICANT:
(A) NAME: ASSOCIATION FRANCAISE CONTRE LES MYOPATHIES
(B) STREET: 13, Place de Rungis
(C) CITY: PARIS
(E) COUNTRY: FRANCE
(F) POSTAL CODE (ZIP): 75013

(ii) TITLE OF INVENTION: METHOD OF REGULATING GENE EXPRESSION
(iii) NUMBER OF SEQUENCES: 11

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:
(i) SEQUENCE CHARACTERISTICS:
(A) LENGTH: 21 base pairs
(B) TYPE: nucleotide
(C) STRANDEDNESS: single
(D) TOPOLOGY: linear

(ii) MOLECULE TYPE: DNA
(iii) HYPOTHETICAL: NO

(xi) SEQUENCE DESCRIPTION: SEQ ID NO: 1:

CCTCCCATAT GTCCAGATTC T 21

(2) INFORMATION FOR SEQ ID NO: 2
(i) SEQUENCE CHARACTERISTICS:
(A) LENGTH: 24 base pairs
(B) TYPE: nucleotide
(C) STRANDEDNESS: single

(D) TOPOLOGY: linear

(ii) MOLECULE TYPE: DNA
(iii) HYPOTHETICAL: NO

(xi) SEQUENCE DESCRIPTION: SEQ ID NO: 2:

CCTTCCTTCT TTTTGATTTT GTTT 24

(2) INFORMATION FOR SEQ ID NO: 3:
(i) SEQUENCE CHARACTERISTICS:
(A) LENGTH: 21 base pairs
(B) TYPE: nucleotide
(C) STRANDEDNESS: single
(D) TOPOLOGY: linear

(ii) MOLECULE TYPE: DNA
(iii) HYPOTHETICAL: NO

(xi) SEQUENCE DESCRIPTION: SEQ ID NO: 3:

CCTCCCATAT GTCCAGATTC T 21

(2) INFORMATION FOR SEQ ID NO: 4:
(i) SEQUENCE CHARACTERISTICS:
(A) LENGTH: 21 base pairs
(B) TYPE: nucleotide
(C) STRANDEDNESS: single
(D) TOPOLOGY: linear

(ii) MOLECULE TYPE: DNA
(iii) HYPOTHETICAL: NO

(xi) SEQUENCE DESCRIPTION: SEQ ID NO: 4:

ACTGCCTCAC CACCGTGCTG G 21

(2) INFORMATION FOR SEQ ID NO: 5:
(i) SEQUENCE CHARACTERISTICS:
(A) LENGTH: 20 base pairs
(B) TYPE: nucleotide
(C) STRANDEDNESS: single
(D) TOPOLOGY: linear

(ii) MOLECULE TYPE: DNA
(iii) HYPOTHETICAL: NO

(xi) SEQUENCE DESCRIPTION: SEQ ID NO: 5:

CAACTTCATC CCGGGGCTCA 20

(2) INFORMATION FOR SEQ ID NO: 6:
(i) SEQUENCE CHARACTERISTICS:
(A) LENGTH: 20 base pairs
(B) TYPE: nucleotide
(C) STRANDEDNESS: single
(D) TOPOLOGY: linear

(ii) MOLECULE TYPE: DNA
(iii) HYPOTHETICAL: NO

(xi) SEQUENCE DESCRIPTION: SEQ ID NO: 6:

CCCTGAGGTA GCATGCGGTA 20

(2) INFORMATION FOR SEQ ID NO: 7:
(i) SEQUENCE CHARACTERISTICS:
(A) LENGTH: 20 base pairs
(B) TYPE: nucleotide
(C) STRANDEDNESS: single
(D) TOPOLOGY: linear

(ii) MOLECULE TYPE: DNA
(iii) HYPOTHETICAL: NO

(xi) SEQUENCE DESCRIPTION: SEQ ID NO: 7:

ATCATGTTTG AGACCTTCAA 20

(2) INFORMATION FOR SEQ ID NO: 8:
(i) SEQUENCE CHARACTERISTICS:
(A) LENGTH: 20 base pairs
(B) TYPE: nucleotide
(C) STRANDEDNESS: single
(D) TOPOLOGY: linear

(ii) MOLECULE TYPE: DNA
(iii) HYPOTHETICAL: NO

(xi) SEQUENCE DESCRIPTION: SEQ ID NO: 8:

TTGCGCTCAG GAGGAGCAAT 20

(2) INFORMATION FOR SEQ ID NO: 9:
(i) SEQUENCE CHARACTERISTICS:
(A) LENGTH: 29 base pairs
(B) TYPE: nucleotide
(C) STRANDEDNESS: single
(D) TOPOLOGY: linear

(ii) MOLECULE TYPE: DNA
(iii) HYPOTHETICAL: NO

(xi) SEQUENCE DESCRIPTION: SEQ ID NO: 9:

CTAGAAAAGG AAAGGAAATA TAACACAGT 29

(2) INFORMATION FOR SEQ ID NO: 10:
(i) SEQUENCE CHARACTERISTICS:
(A) LENGTH: 14 base pairs
(B) TYPE: nucleotide
(C) STRANDEDNESS: single
(D) TOPOLOGY: linear

(ii) MOLECULE TYPE: DNA
(iii) HYPOTHETICAL: NO

(xi) SEQUENCE DESCRIPTION: SEQ ID NO: 10:

AAAGGAAAGG AAAT 14'

(2) INFORMATION FOR SEQ ID NO: 11:
(i) SEQUENCE CHARACTERISTICS:
(A) LENGTH: 15 amino acids
(B) TYPE: amino acid
(C) STRANDEDNESS: single
(D) TOPOLOGY: linear

(ii) MOLECULE TYPE: protein
(iii) HYPOTHETICAL: NO

(xi) SEQUENCE DESCRIPTION: SEQ ID NO: 11:

Phe Arg Arg Gly Thr Gly Gln Ser Asp Asp Ser Asp Ile Trp Asp

                5                   10                  15